# EUROPEAN PATENT SPECIFICATION

(11) **EP 1 118 660 B1**
(45) Date of publication and mention of the grant of the patent: **27.04.2011**
(21) Application number: 99970118.8
(22) Date of filing: 29.09.1999
(51) Int. Cl.: C12N 15/00, C12N 9/50, C07K 14/78, C07K 14/745, C07K 1/22, A61K 38/48, C12N 9/64, C12N 9/68

(54) **ENZYME PRODUCING PLASMA PROTEIN FRAGMENT HAVING EFFECT OF INHIBITING CANCER METASTASIS AND PLASMA PROTEIN FRAGMENT FRAGMENTED BY THE ENZYME**
EIN PLASMAPROTEIN-FRAGMENT ERZEUGENDES ENZYM MIT KREBSMETASTASE-HEMMENDER WIRKUNG UND DURCH DIESES ENZYM FRAGMENTIERTES PLASMAPROTEIN-FRAGMENT
ENZYME PRODUISANT UN FRAGMENT DE PROTEINE PLASMATIQUE AYANT POUR EFFET D'INHIBER LA METASTASE CANCEREUSE ET FRAGMENT DE PROTEINE PLASMATIQUE FRAGMENTE PAR L'ENZYME

(30) Priority: 02.10.1998 JP 29609598
(43) Date of publication of application: 25.07.2001
(73) Proprietor: Juridical Foundation The Chemo-Sero-Therapeutic Research Institute, Kumamoto-shi, Kumamoto 860-8568 (JP)
(72) Inventor: MORIKAWA, Wataru, Kumamoto 862-8003 (JP); KAMINAKA, Kazuyoshi, Kumamoto 861-1102 (JP); TAKEMOTO, Sumiyo, Kumamoto-shi,Kumamoto 861-5522 (JP); MAEDA, Hiroaki, Kumamoto 860-0076 (JP); NOZAKI, Chikateru, Kumamoto 862-8001 (JP); MIYAMOTO, Seiji, Kumamoto 861-1102 (JP)
(74) Representative: Vossius & Partner
(86) International application number: PCT/JP1999/005322
(87) International publication number: WO 2000/020570

(56) References cited:
- EP-A- 0 837 074
- US-A- 5 198 423
- FAUST PH L ET AL: "CLONING AND SEQUENCE ANALYSIS OF CDNA FOR HUMAN CATHEPSIN D" PROCEEDINGS OF THE NATIONAL ACADEMY OF SCIENCES OF USA, NATIONAL ACADEMY OF SCIENCE. WASHINGTON, US, vol. 82, 1 August 1985 (1985-08-01), pages 4910-4914, XP002052494 ISSN: 0027-8424
- CAO ET AL: "KRINGLE DOMAINS OF HUMAN ANGIOSTATIN. CHARACTERIZATION OF THE ANTI-PROLIFERATIVE ACTIVITY ON ENDOTHELIAL CELLS" JOURNAL OF BIOLOGICAL CHEMISTRY, AMERICAN SOCIETY OF BIOLOGICAL CHEMISTS, BALTIMORE, MD, US, vol. 271, no. 46, 15 November 1996 (1996-11-15), pages 29461-29467, XP002086445 ISSN: 0021-9258
- SIM ET AL: "A recombinant human angiostatin protein inhibits experimental primary and metastatic cancer" CANCER RESEARCH, AMERICAN ASSOCIATION FOR CANCER RESEARCH, BALTIMORE, MD, US, vol. 57, no. 7, 1 April 1997 (1997-04-01), pages 1329-1334, XP002100107 ISSN: 0008-5472
- LIJNEN H R ET AL: "Generation of an angiostatin-like fragment from plasminogen by stromelysin-1 (MMP-3)." BIOCHEMISTRY, vol. 37, no. 14, 7 April 1998 (1998-04-07), pages 4699-4702, XP002215765 ISSN: 0006-2960
- MATSUMOTO Y ET AL: "INHIBITORY EFFECT OF ANTIMETASTATIC FUSION POLYPEPTIDE OF HUMAN FIBRONECTIN ON TUMOR CELL ADHESION TO EXTRACELLULAR MATRICES" JAPANESE JOURNAL OF CANCER RESEARCH, JAPANESE CANCER ASSOCIATION, TOKYO, JP, vol. 82, no. 10, October 1991 (1991-10), pages 1130-1138, XP000984063 ISSN: 0910-5050
- SAIKI I ET AL: "INHIBITION OF LUNG METASTASIS BY SYNTHETIC AND RECOMBINANT FRAGMENTS OF HUMAN FIBRONECTIN WITH FUNCTIONAL DOMAINS" JAPANESE JOURNAL OF CANCER RESEARCH, vol. 81, no. 10, 1990, pages 1003-1011, XP001106752 ISSN: 0910-5050
- MORIKAWA WATARU ET AL: "Angiostatin generation by cathepsin D secreted by human prostate carcinoma cells." JOURNAL OF BIOLOGICAL CHEMISTRY, vol. 275, no. 49, 8 December 2000 (2000-12-08), pages 38912-38920, XP002215766 ISSN: 0021-9258
- GATELY S. ET AL.: 'Human Prostate Carcinoma Cells Express Enzymatic Activity That Converts Human Plasminogen to the Angiogenesis Inhibitor, Angiostatin' CANCER RESEARCH vol. 56, no. 21, 01 November 1996, pages 4887 - 4890, XP002925788
- SOFF G. A. ET AL.: 'Expression of Plasminogen Activator Inhibitor Type 1 by Human Prostate Carcinoma Cells Inhibits Primary Tumor Growth, Tumor-associated Angiogenesis and Metastasis to Lung and Liver in an Athymic Mouse Model' J. CLIN. INVEST. vol. 96, no. 6, December 1995, pages 2593 - 2600, XP002925789
- O'REILLY M. S. ET AL.: 'Angiostatin induces and sustains dormancy of human primary tumors in mice' NATURE MEDICINE vol. 2, no. 6, June 1996, pages 689 - 692, XP002925790
- O'REILLY M. S. ET AL.: 'Angiostatin: A Novel Angiogenesis Inhibitor That Mediates the Suppression of Metastases by a Lewis Lung Carcinoma' CELL vol. 79, no. 2, 21 October 1994, pages 315 - 328, XP002925791
- GATELY S. ET AL.: 'The Mechanism of Cancer-mediated Conversion of Plasminogen to the Angiogenesis Inhibitor Angiostatin' PROC. NATL. ACAD. SCI. USA vol. 94, no. 20, September 1997, pages 10868 - 10872, XP002925792
- BRIOZZO P. ET AL.: 'In Vitro Degradation of Extracellular Matrix with Mr 52,000 Cathepsin D Secreted by Breast Cancer Cells' CANCER RESEARCH vol. 48, no. 13, 01 July 1988, pages 3688 - 3692, XP002925793

### SEQUENCE LISTING

<110> The Chemo-Sero-Therapeutic Research Institute
<120> Enzyme Producing Plasma Protein Fragment Having Activity to Inhibit Cancer Metastasis and Cancer Cell Growth and Plasma Protein Fragment Produced by said Enzyme
<130> 661533
<160> 1
<210> 1
   <211> 10
   <212> PRT
   <213> Human Cancer Cell
<400> 1
   Leu Val Arg Ile Pro Leu His Lys Phe Thr
   1               5                   10

## Description

The present disclosure relates to the use of an enzyme that degrades plasma proteins such as plasminogen and fibronectin molecular species into fragments having an inhibitory activity to metastasis and growth of cancer, for producing such plasma protein fragments.

Nowadays, the technique for surgical treatment of cancer has made a remarkable progress and it is estimated that treatment by surgical excision of the primary cancerous focus has almost reached its perfection. However, there still remain many problems to be solved clinically as to a postoperative relapse, metastasis and growth of cancer. Among these, distal metastasis and growth of cancer is a major cause of death in cancer patients.

Metastasis and growth of cancer refers to release of cancer cells from the primary cancerous focus through the vascular system into other sites of the living body where the cancer cells grow. Under the circumstances where cancer cells are spread through the vascular system so that they can grow in a wide range within the living body, it is almost impossible to perfectly remove such cancer cells even with the surgical technique highly progressed nowadays. Thus, in such a case, chemotherapy such as an anti-cancer agent has been used for prophylaxis and treatment of cancer. Even this measure, however, is confronted with the problem of drug resistance to the chemical substance, accompanied by increase in a dose for attaining efficacy of that chemical substance and as a consequence by increase in detrimental side effects. There have been developed a great deal techniques in order to overcome these problems. On the other hand, another approach that is actively pursued by researchers nowadays is to investigate intervention to cancer cells invasion into the vascular system, i.e. intervention to cancer invasion and inhibition of vascularization.

Cancer cells invasion into the vascular system has two aspects: one is related to cancer cells that move from the primary cancerous focus into the vascular system and the other to a drawing-in of the vascular system into the primary cancerous focus. The former is called as cancer cells invasion and the latter as a vascularization. Cancer cells invasion is phenomena in which cancer cells that acquired an ability of metastasis and growth are released from the primary focus (tumor), degrade a barrier between cancer cells and the blood vessel called stroma, and invade into the vascular system (Mignatti P. et al., J. Cell Biol., vol.108, p.671-682 (1989)).

On the other hand, vascularization is phenomena in which novel vessels are formed from the existing vessels. Cancer cells move towards the blood flow through these newly developed vessels. Vascularization is not observed in healthy adult individuals except for sex cycle in females and wound healing but occurs under pathological conditions such as cancer, diabetic retinosis, rheumatism, psoriasis (Folkman J., Adv. Cancer Res., vol. 43, p.175-203 (1985); Zetter B. R., Chest, vol. 93 (Suppl.), p.159S-166S (1998); Patz A., Invest. Ophthalmol. Visual Sci., vol. 19, p.1133-1138 (1980); Amore D. et al., Ann. Rev. Physiol., vol. 49, p.453-464 (1987)). '

Cancer cells (tumor) draw in newly developed vessels from the existing vessels in order to provide them with oxygen or nutrients necessary for their growth. These newly developed vessels facilitate that cancer cells move into the vascular system. Vascularization is a consequence of catabolism of endothelial cells arranged in line within the luminal cavity that are stimulated by cancer cells. Like the invasion process of cancer, multiple steps must occur for development of new vessels after the endothelial cells reached the cancer cells, such as digestion of the basal membrane, release and propagation of the vascular endothelial cells, and formation of luminal cavity. Thus, to intervene to cancer cells invading into the vascular system to inhibit metastasis and growth of cancer is in other words is intervene to any of processes composed of the above multiple steps. Which of these processes should be intervened is disputable but degradation and digestion of the stroma, a common process between cancer invasion and vascularization, is the most highlighted.

### Intervention to Stroma Degradation

Between cancer cells and the vascular endothelial cells, there exist as a barrier to cellular invasion the basal membrane consisted of type IV collagen and the extracellular matrix consisted of collagens. Thus, in order that cancer cells enter the blood flow or the vascular endothelial cells reach cancer cells, this barrier must be digested and degraded (Stetler-Stevenson W. G. et al., Ann. Rev. Cell Biol., vol. 9, p.541-573 (1993)). Enzymes degrading this barrier are a series of enzymes called a matrix-degrading enzyme, including collagenase and gelatinase (Woessner, JF. Jr., FASEB J., vol. 5, p.2145-2154 (1991)). At the site of cancer, the matrix-degrading enzyme is generally overexpressed and excessive expression of serine protease involved in activation of said enzyme is also observed. It is reported that expression of TIMP (Tissue Inhibitor of Metallo Protease) declines at the site of cancer resulting in unbalance between the enzyme and the inhibitor, which provides cancer cells with circumstances for their metastasis and growth (Rak J. et al., Cancer Res., vol. 55, p.4575-4580 (1995)).

Therefore, for normalizing this unbalance, there have been many attempts, for instance, to introduce TIMP-1 gene into cancer cells or to administer a drug that inhibits a matrix-degrading enzyme. Among these are reports in favor of cancer growth inhibition with the use of an inhibitor to gelatinase BB-94 that inhibited experimental metastasis and growth of cancer and intervened vascularization (Davis, B. et al., Cancer Res., vol. 53, p.2087-2091 (1993); Rasmussen, H. S., Proceedings of the ICS 2nd. International conference on protease inhibitors, International business communications, (1997)).

### Relationship between Plasmin and Cancer

A matrix-degrading enzyme is usually present extracellularly in the form of an enzyme precursor (Chen, W. T., Curr. Opin. Cell Biol., vol. 4, p.802-809 (1992)). Thus, there has been an attempt to inhibit degradation of the stroma by inhibiting enzymes involved in activation of this precursor. That is, since a matrix-degrading enzyme is activated through restricted degradation by serine protease, the attempt is concerned with inhibition of said serine protease. A serine protease that activates a matrix-degrading enzyme includes plasmin, urokinase-type plasminogen activator. (hereinafter referred to as "u-PA"), tissue-type plasminogen activator (hereinafter referred to as "t-PA"), trypsin, and the like. Among these, plasmin, an enzyme associated with blood fibrinolysis, is highlighted in that it is a common enzyme activated both by cancer cells and vascular endothelial cells. Specifically, cancer cells have an ability to produce u-PA whereas vascular endothelial cells to produce t-PA wherein plasmin is a consequence of activation of its precursor plasminogen through cleavage by the action of a plasminogen activator such as u-PA and t-PA. It is reported that both u-PA and t-PA are expressed at a high level under cancerous conditions.

In addition to activation of a matrix-degrading enzyme as mentioned above, plasmin also has an ability to activate TGF-β, a factor involved in vascularization, and an ability to degrade the extracellular matrix (Werb Z. et al., N. Engl. J. Med., vol. 296, p.1017 (1977); Brunner G. et al., J. Cell Biol., vol. 6, p.1275-1283 (1991)). Moreover, it is considered that plasmin has an ability to destroy protective mechanism of hosts that besieges cancer with fibrin membrane to thereby suppress cancer dispersion.

Taking the above facts into consideration, it is supposed that plasmin expression is rather inclined to promote cancer invasion at the sites where cancer exists. Supporting this supposition, it is indeed reported that an inhibitor to plasmin did inhibit metastasis and growth of cancer cells in experimental animals (Ohta T. et al., Cancer and Clinics, vol. 23, p.1669-1672 (1996); Ohkoshi M., Cancer and Chemotherapy, vol. 22, p.417-430 (1995)).

### Inhibitory Activity to Metastasis and Growth of Cancer by Angiotensin-like Molecule

On the other hand, it is reported that products produced by fragmentation of plasminogen and plasmin exhibit an inhibitory activity to growth or invasion of cancer cells. That is, a report on an antiangiogenic factor angiostatin (O'Reilly M. S. et al., Cell, vol. 79, p.315-328 (1994)). Angiostatin is a vascularization-inhibitory substance comprising an inner fragment of plasminogen and has a potent inhibitory activity to vascularization and growth of metastasized cancerous focus at an extremely low amount. It also possesses, amazingly, an ability to induce recession of cancer (primary focus) in a dose dependent manner without any side effects or drug resistance (O'Reilly M. S. et al., Nat. Med., vol. 2, p.689-692 (1996)). Angiostatin has been correlated with inhibition to vascularization and cancer as demonstrated by O'Reilly et al. in sophisticated experiments. However, most of mechanism of inhibitory action of angiostatin and its production still remains to be elucidated.

The above teaching suggests a hypothesis that plasmin production leads to metastasis and growth of cancer whereas fragmentation of plasminogen or plasmin leads to inhibition of metastasis and growth of cancer. Supposing that this balance is broken down to be inclined to metastasis and growth of cancer under cancerous conditions, it would be possible to inhibit metastasis and growth of cancer if such an unbalance could be shifted to fragmentation of plasminogen or plasmin. That is, it is supposed that there might exist an enzyme that cleaves plasminogen or plasmin and produces angiostatin-like molecules as a mechanism of the living body to protect against cancer development. The present inventors, in order to confirm the genuineness of the supposition, have investigated an enzyme that cleaves plasminogen or plasmin to produce the angiostatin-like molecules.

The present inventors have earnestly investigated in order to solve the above problems and as a result have found a novel enzyme activity in a culture of PC-3, prostate cancer cell line, said enzyme activity being able to fragment plasminogen only under low pH condition. It was found that plasminogen fragments produced by said enzyme comprises plasminogen Kringles 1 to 4 and that said enzyme inactivates plasmin by cleaving it in the vicinity of the active center. This activity is expressed in most of cancer cells and hence suggested to be specific to cancer.

Purification with various chromatographs revealed that this enzyme had a molecular weight of about 45 kDa. It was also found that an N-terminal amino acid sequence of this enzyme was initiated with LVRIPLHKFT which had a high homology to Human Cathepsin D Precursor. Investigation with inhibitors revealed that this enzyme was classified into an aspartic enzyme. The present inventors designated this enzyme as "PACE4" (Plasminogen Angiostatin Converting Enzyme of pH 4) in connection with its exertion of the activity at a lower pH.

Moreover, the enzyme purified from supernatant of PC-3 culture was reacted with plasminogen to produce plasminogen fragments that were then administered to a mouse model in which Lewis lung cancer was transplanted. This confirmed that the obtained plasminogen fragments had an inhibitory activity to metastasis and growth of cancer cells. Also, various plasma components were cleaved with this enzyme and the obtained fragments were screened for their inhibitory activity to vascularization. As a result, in addition to plasminogen, it was revealed that fibronectin and vitronectin, both components involved in cell adhesion, and human hepatocyte growth factor (HGF) were also fragmented by this enzyme and that plasma protein fragments produced by fragmentation had a potent inhibitory activity to vascularization.

Thus, the present invention relates the use of an aspartic enzyme as defined in the claims.
Fig. 1 indicates fragmentation of plasminogen when supernatant of PC-3 culture was reacted with plasminogen at various pHs.
Fig. 2 illustrates a scheme of assays for determining the plasminogen-fragmenting enzyme.
Fig. 3 shows elution patterns when supernatant of PC-3 culture was subjected to hydrophobic chromatography.
Fig. 4 shows SDS-PAGE of purified PACE.
Fig. 5 shows results obtained after reaction of the purified PACE4 with various protease inhibitors to investigate a mode of inhibition of this enzyme.
Fig. 6 shows cleavage patterns obtained after reaction of the purified PACE4 with plasminogen either at pH 7.0 or pH 4.0.
Fig. 7 shows cleavage patterns with passage of time when plasminogen (Glu-1) is cleaved with PACE4.
Fig. 8 shows disappearance of plasmin activity by PACE4.
Fig. 9 shows an inhibitory activity of PACE4 to growth of metastasized cancerous focus.
Fig. 10 shows investigation as to production of PACE4 from various cancerous cells wherein the PACE4 activity is indicated as an amount of degraded plasminogen.
Fig. 11 shows investigation on plasminogen degradation by PC-3. (A): SDS-PAGE of plasminogen fragments produced by PC-3; (B): Western blot with anti-Mini Plasminogen antibody and anti-LBS-I antibody.
Fig. 12 compares a cleavage rate of Glu-plasminogen and Lys-plasminogen by PACE4.
Fig. 13 shows electrophoretogram of fragments comprising Kringles 1 to 4 produced by PACE4.
Fig. 14 shows electrophoretogram of a reaction mixture after PACE4 is contacted with fibronectin under various conditions in connection with cleavage of fibronectin by PACE4.
Fig. 15 shows results obtained after reaction of products produced by fragmentation of fibronectin by PACE4 with vascular endothelial cells.

### Finding of Said Enzyme Activity

A preferential measure for investigating an enzyme that produces an angiostatin-like molecule and inactivates plasmin is to find out an enzyme that produces angiostatin. As already reported, angiostatin has been found in plasma and urine of a mouse transplanted with Lewis lung cancer subspecies 3LL-LM and hence an enzyme that produces angiotensin should exist in said mouse. O'Reilly et al. made a model using 3LL-LM cells with perception of rapid growth of distally metastasized cancerous focus that is rarely observed after excision of malignant tumor and found in plasma and urine of the model a potent inhibitory factor to vascularization, leading to discovery of angiostatin. Under cancerous conditions, as they say, a factor derived from a primary focus (cancer) is circulating throughout the blood stream to thereby inhibit growth of a distally metastasized cancerous focus. Also, as far as angiostatin is concerned, it is estimated that since cancer cells do not produce plasminogen, an enzyme derived from cancer cells cleaves plasminogen occurring in blood to thereby produce angiostatin.

Various candidates for an enzyme that produces angiostatin have been reported from several laboratories. In recent years, Gately et al. reported that the activity to produce angiostatin through cleavage was present in culture supernatant of human prostate cancer cells (PC-3 cells) and designated an enzyme that provides this activity as PACE (Plasminogen Angiostatin Converting Enzyme) (Gately S. et al., Cancer Res., vol. 56, p.4887-4890 (1996)). PC-3 cells are such human type cells that have property that excision of primary cancerous focus can conversely promote growth of distally metastasized cancerous focus (Soff G. A. et al., J. Clin. Invest., vol. 96, p.2593-2600 (1995)) and hence human angiostatin and angiostatin-producing enzyme (PACE) produced by said cancer cells are noticeable. Moreover, they prepared a plasminogen fragment by reaction of plasminogen with PC-3 culture supernatant and demonstrated that this fragment had an inhibitory activity to vascularization equivalent to that of angiostatin reported by O'Reilly et al. both in vitro and in vivo.

There have also been reports by Dong et al. and by Brain et al. Dong et al. focused on macrophage invasion observed under cancerous environments and revealed that an enzyme derived from macrophage (matrix metallo elastase) was an enzyme that produced angiostatin (Dong Z. et al., Cell, vol. 88, p.801-810 (1997)). Brain et al. screened Matrix metallo proteinase (MMP) to thereby reveal that MMP-7 (also referred to as "Matrilysin") and MMP-9 (also referred to as "Gelatinase B") exhibited the activity to produce angiostatin (Brain C. et al., J. Biol. Chem., vol. 272, p.28823-28825 (1997)).

At that time, however, PACE according to Gately et al. was identified merely for its activity but the enzyme per se had not yet been isolated and established. Therefore, the present inventors firstly conducted the experiments according to Gately et al. aiming at isolation and purification of PACE. When PC-3 culture supernatant prepared in accordance with Gately et al. was reacted with plasminogen, a plasminogen fragment corresponding to angiostatin was indeed confirmed to exist at 50 kDa on SDS-PAGE. However, the cleavage pattern was poorly specific and at least it was not assured that PACE specifically cleaved plasminogen.

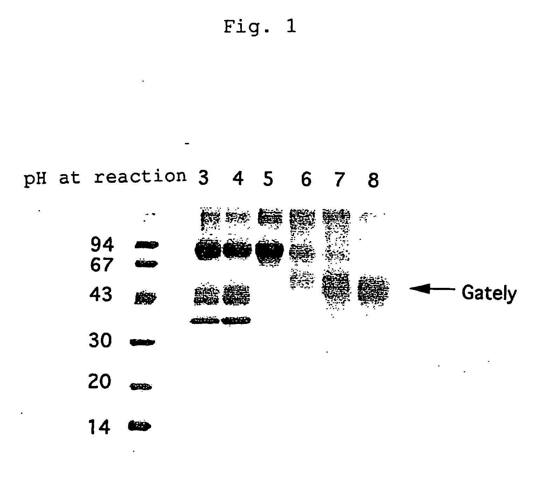

The present inventors have constructed a screening system of our own with which we tried to isolate and purify the desired enzyme. As a result, of thorough investigation, the present inventors have successfully found, in addition to an enzyme that fragments plasminogen at neutral pH, that an enzyme activity that can specifically cleave a restricted site of plasminogen under lower pH condition was present in PC-3 culture supernatant. This enzyme activity has not been reported previously and is utterly different from PACE according to Gately et al. The cleavage pattern of this enzyme proved, as shown in Figure 1, that it specifically cleaved a restricted site of plasminogen unlike PACE that nonspecifically cleaved plasminogen.

The present inventors have paid attention to lower pH at the cancerous state since previously and reported possibility that some plasminogen fragments produced by cleavage through elastase activity might accumulate at the site with lower pH (The 56th General Meeting of Japan Association of Oncology, excerpt, p.426, 1997). The present inventors have already found that an enzyme that functions at that lower pH was related to cancerous conditions by investigating enzyme activities with various types of cancer cells and normal cells and perceived that said enzyme was produced in a large amount only under cancerous conditions through preliminary investigation. As mentioned above, a purpose of the present inventors' screening is to prove the hypothesis that "an enzyme that cleaves plasmin to thereby produce angiostatin-like molecules that can do away with the plasmin activity should have been provided in the living body as a protective mechanism to cancer development". It is highly possible that it is this enzyme functioning only under lower pH conditions what is referred to in said hypothesis. Thus, the present inventors have initiated investigation for isolation and purification of said enzyme.

Recently, Gately et al. succeeded in isolation of the PACE enzymes per se and reported that these enzymes were a consequence of plasmin and free cysteine donors (Gately S. et al., Proc. Natl. Acad. Sci., vol. 94, p.1068-1087 (1997)).

### Investigation of Enzyme of the Present Invention

The most characteristic property of the enzyme of the present invention is its ability to specifically cleave plasma proteins such as plasminogen and fibronectin at a restricted site. The present inventors have firstly initiated investigation and isolation of the enzyme in order to elucidate relationship between said enzyme and cancer cells.

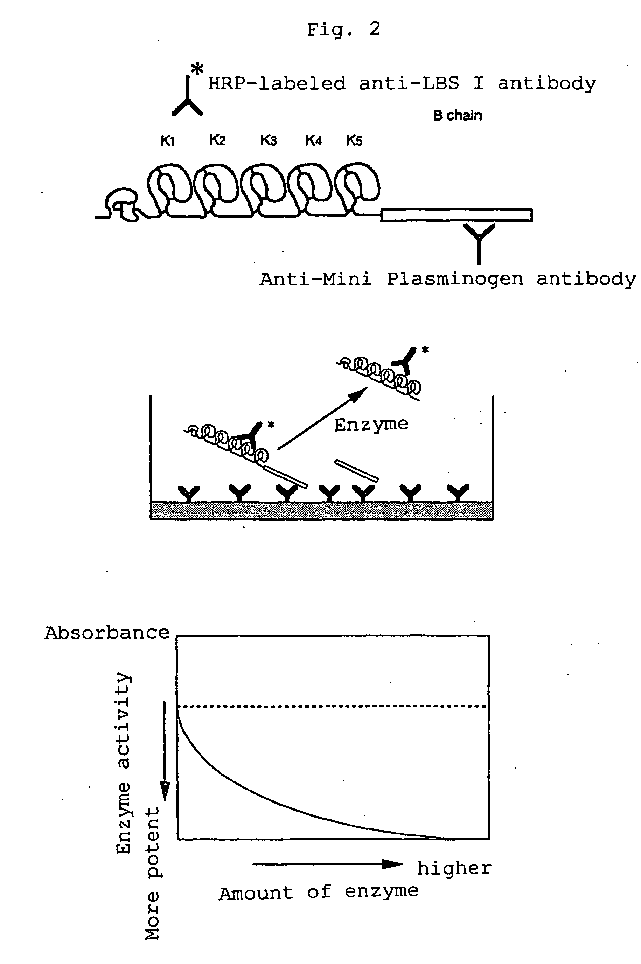

The present inventors have first constructed a screening system that enables detection of a rate at which plasminogen is fragmented at a high sensitivity. With this screening system, a combination of various chromatographs could purify the desired enzyme. Fig. 2 schematically illustrates the screening system for the plasminogen-fragmenting enzyme. This method is a kind of modified sandwich ELISA wherein a specific antibody to Plasminogen Lysine Binding Site I (hereinafter referred to as "LBS I") is used as an immobilized antibody whereas a specific antibody to Mini Plasminogen (hereinafter referred to as "mPlg.") is used as a labeled antibody. When an objective enzyme is contacted with a predetermined amount of plasminogen, plasminogen is cleaved to thereby lose a region that is recognized by the labeled antibody and as a consequence color development in ELISA declines. The enzyme activity is estimated by detecting this decline of color development.

### Preparation of Enzyme of the Present Invention

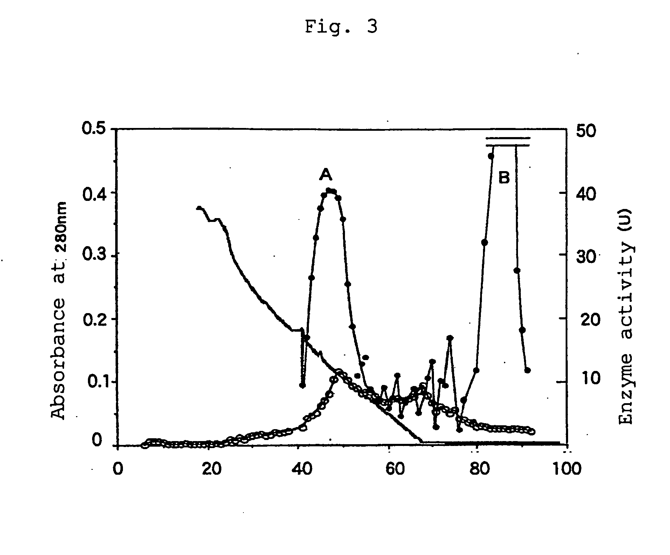

Preparation of the desired enzyme was achieved, using PC-3 culture supernatant as starting material, by a series of chromatographic procedures including a cation exchanger, a heparin chromatography, an anion exchanger, gel filtration, and hydroxyapatite. The first procedure with a cation exchanger was carried out primarily for the purpose of removing contaminating single chain urokinase-type plasminogen activator (scu-PA) whereas heparin chromatography was used for removal of serine protease. Scu-PA, a precursor of plasminogen activator, was activated by the presence of plasmin into an active form of u-PA, which also cleaves plasminogen at a neutral pH range. Thus, it is absolutely necessary to remove scu-PA at an early stage for purification of the desired enzyme. The enzyme of the present invention is well isolated by hydrophobic chromatography. Fig. 3 shows a chromatogram of typical hydrophobic chromatography wherein the open circle indicates absorbance, the closed circle indicates an enzyme activity, and the wave line indicates an ionic strength. Fig. 3 clearly indicates two distinct enzyme activities with different hydrophobicity in PC-3 culture supernatant. Among these, the enzyme activity that specifically cleaved plasminogen to produce plasminogen fragments comprising Kringles 1 to 4 was detected in the peak with higher hydrophobicity (at right side of chromatogram). The enzyme activity in the peak with lower hydrophobicity (at left side of chromatogram) fragmented plasminogen nonspecifically and, as a result of isolation and purification, was found to be a protein having high homology with cathepsin E.

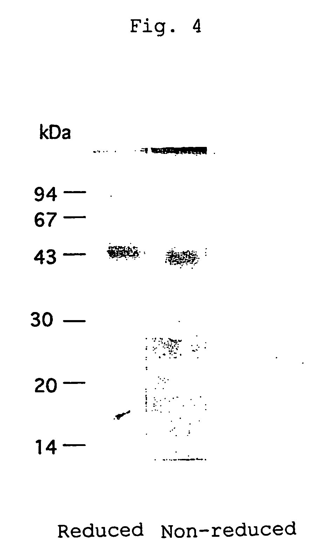

Isolation with gel filtration chromatography was performed for examining a molecular weight of the enzyme of the present invention, and as a result, the enzyme activity was recovered at around 50 to 60 kDa. Fig. 4 shows results of SDS-PAGE analysis of the enzyme of the present invention wherein a band with the activity was found at the position corresponding to 45 kDa of a molecular weight. Since it was revealed that the enzyme of the present invention was an aspartic enzyme as described below, it was purified in the end by affinity chromatography using pepstatin as a ligand.

The enzyme of the present invention may be prepared, in addition to the procedure as described above starting from PC-3 culture supernatant, by constructing cells that produce said enzyme with the genetic recombinant technique and recovering said enzyme produced by said cells. That is, the enzyme of the present invention may also be prepared by an approach wherein a gene encoding said enzyme is introduced with the aid of an appropriate vector into an appropriate host including prokaryotic, eukaryotic, mammal or insect cells, from which the enzyme is recovered.

### Property of the Enzyme of the Present Invention

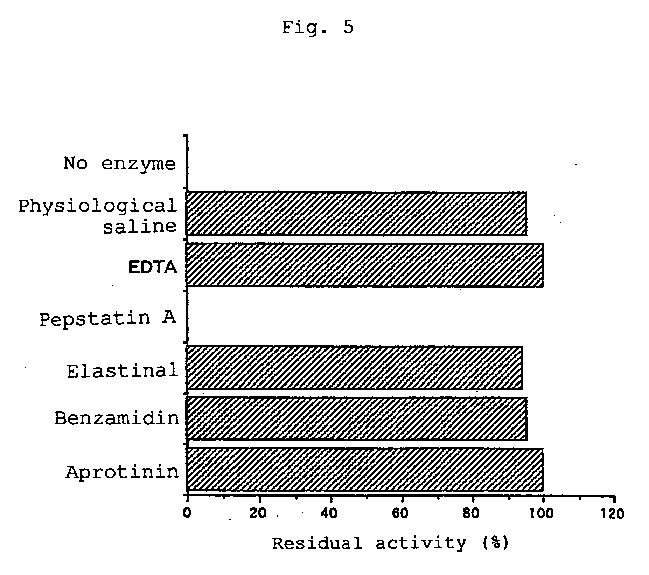

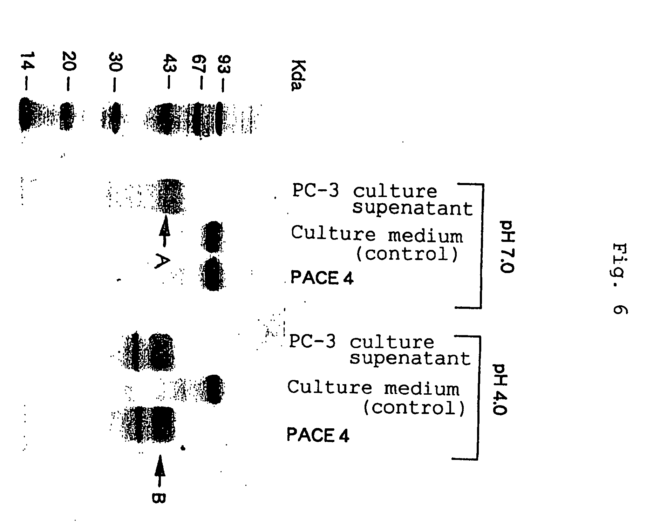

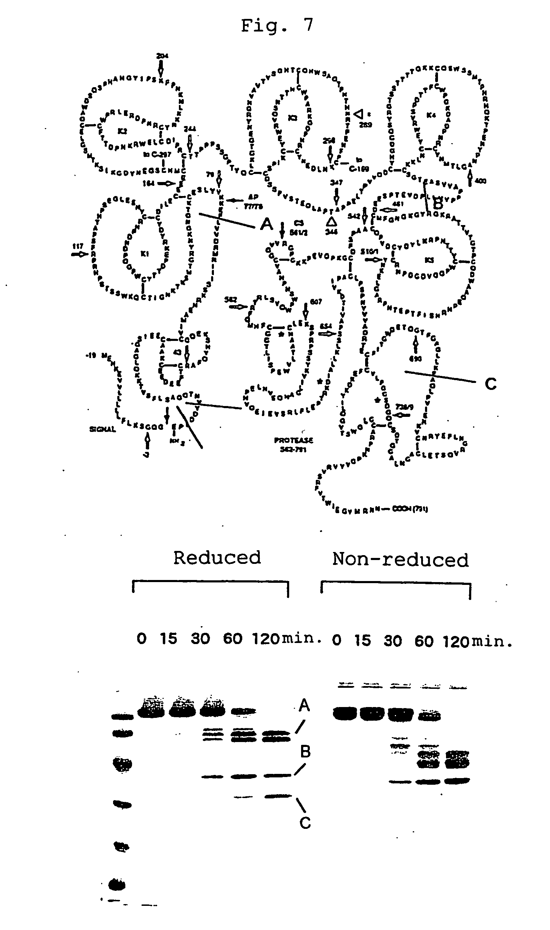

A mode of the inhibitory activity of the enzyme of the present invention is summarized in Fig. 5 wherein a concentration of each inhibitor was: aprotinin (0.3 µM), benzamidine (10 mM), elastinal (100 µM), pepstatin A (1 µM), and EDTA (10 mM). As shown in Fig. 5, the activity of the enzyme of the present invention was completely inhibited by pepstatin A, a specific inhibitor to an aspartic enzyme. Fig. 6 depicts fragmentation patterns of plasminogen when reacted at neutral and acidic ranges of pH. As is clearly shown, the enzyme of the present invention fragments plasminogen only at an acidic range of pH. In Fig. 6, the arrow A (→A) indicates a band corresponding to angiostatin from PC-3 according to Gately et al. whereas the arrow B (→B) indicates a band corresponding to a fragment comprising Kringles 1 to 4 produced by PACE4. Fig. 7 shows sites where the enzyme of the present invention cleaves plasminogen, which is cleaved into A (plasminogen Kringles 1 to 4), B (Mini Plasminogen) and C (Mini Plasminogen fragments). The N-terminal amino acid residue of each fragment was analyzed to be F-74 for A, P-452 for B and A-700 for C, respectively.

The enzyme of the present invention cleaves plasminogen at between the 451st, from the N-terminus, Leu and the 452nd Pro (hereinafter referred to as "451L-P"), and between the 73rd Leu and the 74th Phe (hereinafter referred to as "73L-F") to leave a fragment comprising Kringles 1 to 4 of plasminogen. Plasminogen circulates within the living body mostly in the form not cleaved and has the N-terminal Glu (intact plasminogen). However, a part of plasminogen, several percent, is cleaved at the N-terminal and has the N-terminal 78 Lys, called Lys plasminogen. The enzyme of the present invention, in addition to intact plasminogen, fragments Lys plasminogen as well and thus plasminogen fragments produced by the enzyme of the present invention include one with the N-terminal amino acid being 78 Lys.

Moreover, the enzyme of the present invention, producing angiostatin that comprises Kringles 1 to 4, also cleaves plasmin in the vicinity of its active center (between the 699th Phe and 700th Ala) to let plasmin be inactivated. This cleaving property of the enzyme is quite significant in view of efficient protection against cancer development. This is because angiostatin that inhibits growth of cancer is produced by fragmentation of plasminogen while the simultaneously produced active center of plasmin (plasmin serine protease domain) possibly further promotes cancer development. In addition, it is possible that vascularization and cancer invasion accelerated by the plasmin activity is further accelerated by removal of the Kringle domains. This is because such removal of the Kringle domains would attenuate the inhibitory activity of plasmin-inhibitory proteins occurring within the living body, including alpha 2 plasmin inhibitor (hereinafter referred to as "α2-PI"). Plasmin present in plasma is immediately inactivated by α2-PI circulating in the blood stream wherein the Kringle domains serve as a binding site between plasmin and α2-PI. Deprivation of the domains much reduces the activity of α2-PI. Thus, the active plasmin fragment free from inhibition may spread to a cancerous region where it activates a matrix-degrading enzyme to thereby accelerate cancer development and metastasis and growth of cancer cells.

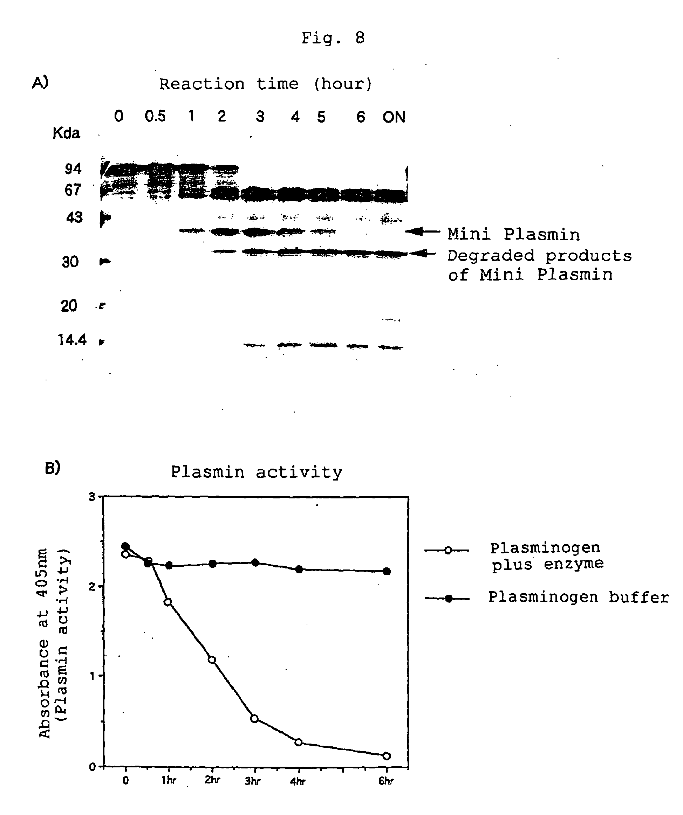

The enzyme of the present invention is distinct from the previously reported enzymes that produce angiostatin in that it not only produces fragments comprising Kringles 1 to 4 of plasminogen but also inactivates the remaining plasmin activity. Supposing whatever protective role in the living body the enzyme of the present invention plays, it is probably a top-rated candidate enzyme. Fig. 8 shows a relationship between plasminogen cleavage by the enzyme of the present invention with passage of time and the remaining plasmin activity: (A) SDS-PAGE when plasminogen is fragmented with PACE4; (B) the plasmin activity measured for the samples. Fig. 8 clearly indicates that the plasmin activity disappears as mini plasminogen is more cleaved. This result suggests that the enzyme of the present invention can function in favor of the living body and hence it is expected that a substrate protein to which the enzyme of the present invention acts is not limited to plasminogen. The present inventors, in order to verify this possibility, also performed fragmentation of other plasma proteins as described below and could successfully prepare fragments with the activity equivalent to or even exceeding ones from plasminogen using the enzyme of the present invention.

### Identification of the Enzyme of the Present Invention

After the purified enzyme sample was electrophoresed on SDS-PAGE, it was transferred to GVDF membrane by blotting. The blotted membrane was dyed with Amido Black, bands corresponding to 45 kDa were excised and the N-terminal amino acid sequence was read with an amino acid sequence analyzer. As a result, the band corresponding to 45 kDa had a sequence LVRIPLHKFT. The determined amino acid sequence was compared with the existing amino acid data bank and was found to have homology with a precursor of human cathepsin D. When the enzyme purified by immunoblot was reacted with an anti-human cathepsin D antibody, said enzyme responded to this antibody. Thus, it is estimated that the enzyme of the present invention has a high homology with human cathepsin D.

Cathepsin D is produced as cathepsin D precursor having a whole amino acid sequence which is then cleaved by other enzymes in lysosome into active cathepsin D. There are many reports that a malignant tumor, typically human breast cancer, excretes the cathepsin D precursor out of cells. Supposing that the enzyme of the present invention is the cathepsin D precursor, it is just a novel and interesting finding that the cathepsin D precursor in the form of a precursor does cleave plasminogen.

cDNAs were synthesized from mRNAs prepared from PC-3 cells that were used as starting material for preparing PACE4. Sense and antisense primers for amplifying a whole translation region of cathepsin D were synthesized on the basis of cDNA of cathepsin D. With the cDNAs from PC-3 cells, the sense and antisense primers were used for amplification with AmpliTaq (PERKIN ELMER) and the amplified cDNA fragment was cloned into a plasmid vector with TA cloning kit (Invitrogen). The obtained gene fragment was sequenced to confirm that it was identical to the known nucleotide sequence of cathepsin D. If PACE4 is identified as the cathepsin D precursor, however, some queries arise why an inactive form precursor can cleave, say, plasminogen, or why cathepsin, that is an intracellular enzyme, is excreted out of cells. For proving that the enzyme of the present invention is genuinely identical to the cathepsin D precursor, a whole amino acid sequence as well as a structure of the enzyme remain to be elucidated. At present, both cathepsin D precursor and active cathepsin D have the enzymatic activity of the present enzyme and are encompassed by PACE4. Also, derivatives of cathepsin D with deletion, substitution or chemical modifications are included within a scope of the enzyme of the present invention insofar as they are used for the purpose of preparing plasma protein fragments having an inhibitory activity to metastasis and growth of cancer.

### Preparation of Protein Fragments after Cleavage with PACE4

Plasminogen and PACE4 are mixed at a predetermined ratio and reacted at pH 4.0. After dialysis with a buffer at a neutral pH range, the reaction mixture is contacted with lysine Sepharose so that fractions containing a fragment comprising Kringles 1 to 4 of plasminogen are absorbed to the resin and separated based on binding capacity to lysine. The obtained fragments are lyophilized after dialysis with an ammonium carbonate buffer. Fibronectin may similarly be cleaved and a fragment mixture is subjected to heparin Sepharose separation to prepare a desired fragment.

### Use of PACE4

### 1. Use of protein fragments obtained after cleavage with PACE4 as a medicament for inhibiting growth of metastasized cancerous focus

The protein fragments prepared as described above were dissolved in a physiological saline and used as a sample for animal tests. As a system for estimating an inhibitory effect to growth of metastasized cancerous focus, cancer capable of metastasis and growth was transplanted to mice where distally metastasized cancerous focus was observed. Lung cancer cells (LL/2; Dainippon Seiyaku K.K.) were subcutaneously transplanted to mice at the back. The animals were bred until a primary cancerous focus grows up to a predetermined size and the primary focus was then removed surgically. The sample was administered to mice intraperitoneally at 1 mg/mouse/day for 10 days. As a control, mice were administered with a physiological saline alone as used for dissolving the lyophilized sample (test for inhibitory activity to growth of metastasized cancerous focus).

For test for inhibitory activity to metastasis and growth of cancer, after removal of the primary cancerous focus, mice were bred for additional predetermined period of time and then administered with the sample at 1 mg/mouse/day for 10 days.

After completion of each test, mice were dissected. The number of nodes on the lung surface, in case of test for inhibitory activity to metastasis and growth of cancer, or a weight of the lung, for test for inhibitory activity to growth of metastasized cancerous focus, was measured respectively and compared with control groups (Mann Whetney U test). As a result of test for inhibitory activity to metastasis and growth of cancer, the number of nodes representing metastasis and growth of cancer was 6.3 ± 0.2 g (n=6) for the control group while it was 2.1 ± 0.8 g (n=6) for the group administered with the plasminogen fragment. Thus, it was proved that metastasis and growth of cancer was inhibited by administration of the plasminogen fragment. In test for inhibitory activity to growth of metastasized cancerous focus, the lung was measured 0.44 + 0.28 g (n=6) for the control group while it was measured 0.23 ± 0.08 g (n=6) for the group administered with the plasminogen fragment. Thus, it was proved that growth of LL/2 cells after metastasis and growth of cancer to the lung was inhibited by administration of the plasminogen fragment.

It is estimated that the above results reflect inhibition of metastasis and growth as well as inhibition of vascularization. Inhibition of metastasis and growth is estimated to be a consequence of various direct and indirect inhibitory processes including inhibition of matrix-degrading enzyme activation by the fragment, inhibition of the plasmin activity through competitive inhibition of plasmin, and down regulation of matrix-degrading enzyme via the receptor. On the other hand, the effect to vascularization is estimated to be a consequence of the activity of the plasminogen fragment produced by the fragmenting activity of the present enzyme, said activity of the fragment being equivalent to that of angiostatin and Kringles of plasminogen reported by O'Reilly et al. However, in a test for inhibitory activity to growth of the endothelial cells using the vascular endothelial cells of bovine aorta, inhibitory effects to its growth have not been obtained. Thus, mechanism of inhibition of growth of metastasized cancerous focus still remains to be elucidated.

### 2. Use of PACE4 as a medicament for inhibiting growth of metastasized cancerous focus

As a system for estimating inhibitory effects to growth of metastasized cancerous focus of PACE4 per se, immunodeficient (Scid) mice were employed. Lung cancer cells (3LL; provided from Cell Resource Center for Biomedical Research, Institute of Development, Aging and Cancer, Tohoku University) were subcutaneously transplanted to Scid mice at the back. The animals were bred until a primary cancerous focus grows up to a predetermined size. So that severity of disease conditions is taken into consideration, mice were divided into two groups based on the size of a primary cancerous focus; one with a primary cancerous focus weighing not more than 1200 mg and the other with a primary cancerous focus weighing more than 1200 mg. The primary cancerous focus in each group was removed surgically. Each group was then further divided into three groups: a group receiving a high or low concentration of PACE4, and a control group. The sample was administered to mice intraperitoneally at 40 µg/mouse/day or 8 µg/mouse/day for 10 days. As a control, the same amount of physiological saline, as used for dissolving the lyophilized sample, was administered to mice.

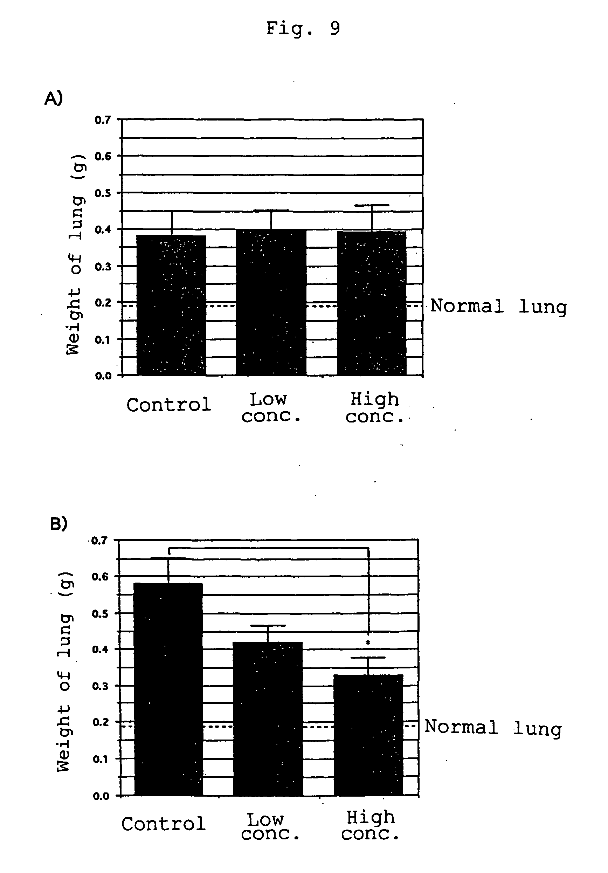

After completion of test, mice were dissected and the lung was weighed and compared with the control group (Mann Whetney U test). As a result, most interestingly, the enzyme of the present invention exhibited inhibitory activity to growth of metastasized cancerous focus in a concentration dependent manner in the group of a primary cancerous focus weighing more than 1200 mg (B) (Fig. 9).

The above results are quite interesting since that the cathepsin D-like enzyme of the present invention functions within the living body implies that there exists an environment with low pH, at least pH 5.0. Such an environment with pH of as low as 5.0 is not physiologically observed. At present it has not yet been elucidated how this enzyme functions physiologically but there is also a report that a cancerous environment provides this low pH environment. Briozzo et al. reported that cathepsin D, which is an aspartic enzyme classified into the same class as the enzyme of the present invention, was secreted out of cancer cells and acted at distal sites where it degraded the extracellular matrix (Briozzo et al., Cancer Res., vol. 48, p.3688-3692 (1988)). This shows that in the stroma there is a low pH region of less than pH 5.5 where an aspartic protease can function. This low pH is particularly observed in a microenvironment produced by malignant tumor residing adjacent to the extracellular matrix. The cause of this is considered to be shift to acidic side at the cellular membrane (Carrel A. et al., J. Exp. Med., vol. 144, p.503-521 (1976)) due to increase in proton released from cancerous membrane proteins or increase in H-ATPase (Baron R. et al., J. Cell Biol., vol. 101, p.2210-2222 (1995)) or a sialic acid content of oligosaccharide chain of cancer cells (van Beek W. P. et al., Cancer Res., vol. 33, p.2913-2922 (1973). It has also been proposed that acidification in cancer was a consequence of anaerobic condition (Spechler S. J. et al., Arch. Int. Med., vol. 138, p.1663-1664 (1978)). Since both cathepsin D and the enzyme of the present invention have a membrane-binding ability, it is conjectured that they are released out of cells while being encapsulated with membrane. Intracellular proteases such as cathepsin D are primarily localized in membranous organelle such as endosome and lysosome. Both of these membranous organelle are extremely shifted to acidic side due to distribution of H-ATPase and play a role in degradation and reuse of intracellularly or extracellularly existing heterogeneous substances. It is also conjectured that a certain condition is loaded to cancerous cells by some reasons to release such membranous organelle out of the cells, which in turn digest plasminogen or the extracellular matrix neighboring said membranous organelle.

### 3. Degradation of Plasma Proteins such as Fibronectin by PACE4

The present inventors focused on fibronectin, a plasma component that is produced in great deal under cancerous conditions and is deeply involved in cell adhesion, and cleaved this with the enzyme of the present invention. Fibronectin was degraded restrictedly with this enzyme and its degraded products were found to have a potent inhibitory ability to BCE (Bovine capillary endothelial) vascular growth not found in intact fibronectin. The degraded products produced by the enzyme from fibronectin were separated with heparin Sepharose (Pharmacia). A fraction of a heparin-binding activity was administered to mice of the model system as described above at 1 mg/kg/day to prove that this fragment had an inhibitory activity to growth of metastasized cancerous focus. This result apparently indicates that a source of such a fragment that inhibits growth of metastasized cancerous focus is not limited to plasminogen. In order to prove this hypothesis, the present inventors reacted the enzyme of the present invention with tetranectin, a component of the matrix, or plasma proteins having similar Kringle domains as plasminogen. The obtained degraded products were estimated for their inhibitory activity to vascularization in the vascular endothelial cell system as described above.

Moreover, the present inventors also cleaved several kinds of fractions obtained from alcoholic fractionation of plasma proteins with the enzyme of the present invention. Alcohol was removed from alcoholic fractions by dialysis, followed by dialysis with citric phosphate buffer (pH 4.0). The resulting fractions were mixed with the enzyme at a ratio of the protein to the enzyme, 100:1, and the mixture was reacted at 38°C overnight. The crude solutions of the protein may form a lot of precipitates at 4°C and hence were centrifuged at 6000 rpm (manufactured by Tommy) prior to the enzymatic reaction and their supernatant was used. The reaction solutions were dialyzed against a 50 mM Tris/50 mM NaCl (pH 7.2) buffer overnight, filtered with a 0.45 µm filter (Milex HA: manufactured by Millipore) and treated with 5 ml of heparin Sepharose (Hi-trap Heparin: manufactured by Millipore) to give protein mixtures. Although only with activity at present, the present inventors cleaved fraction III or fraction IV from alcoholic fractionation with the enzyme and found, in those fractions that bound to the heparin carrier, an inhibitory activity to growth of the vascular endothelial cells.

### 4. Measurement of PACE4 in plasma from patients suffering from cancer

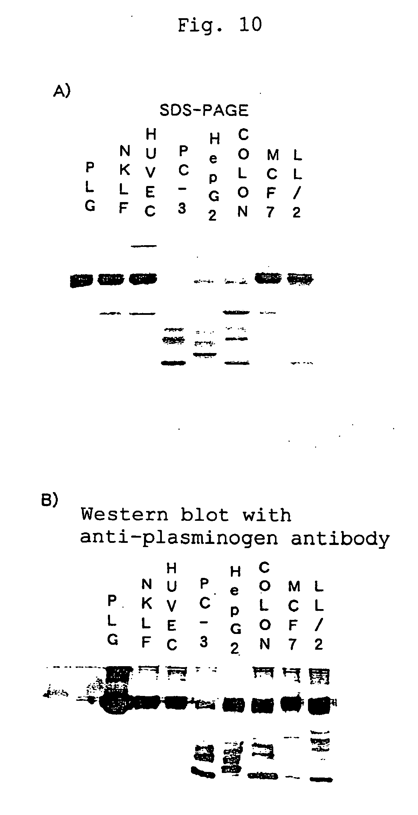

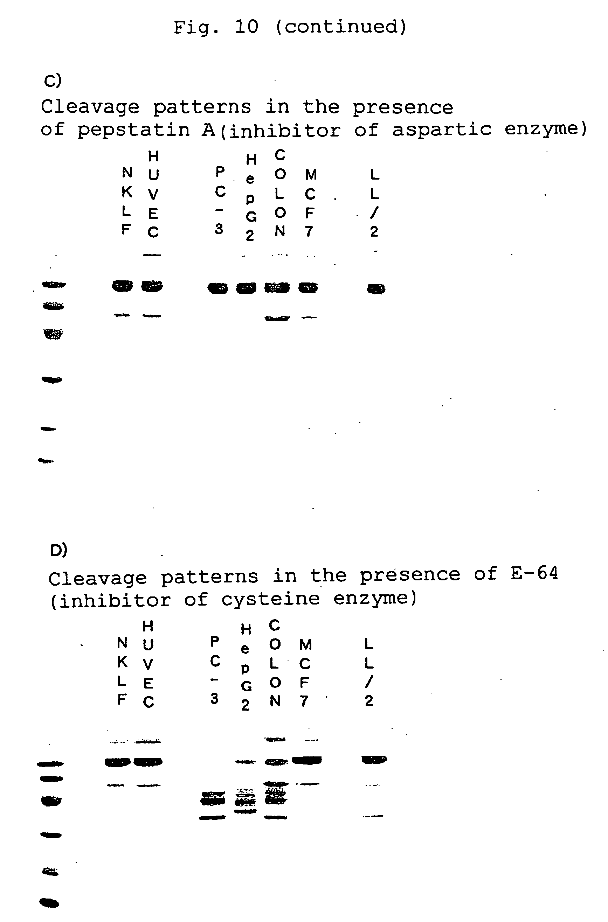

PACE4 activity in plasma from patients suffering from cancer was measured as described for measurement of PACE4 activity in PC-3 culture supernatant. Test samples were subjected to measurement of PACE4 wherein enzymatic activities released out of various cancer cells into culture supernatant were measured by detecting the presence of the plasminogen-degrading activity as described above. As a result, it was revealed that, in normal cells, PACE4 was not released out of cells but a great deal of PACE4 was released out of some cancerous cells (Fig. 10). In Fig. 10,(A) SDS-PAGE; (B) Western blot using anti-plasminogen antibody; (C) SDS-PAGE when reacted in the presence of inhibitors to PACE4; and (D) SDS-PAGE when reacted in the presence of inhibitors to cysteine enzymes. Also in Fig. 10, PLG: plasminogen; NKLF: smooth muscle cells; HUVEC: vascular endothelial cells; PC-3: prostate cancer cells; HepG2: hepatic cancer cells; COLON: colon cancer cells; MCF7: breast cancer cells: LL/2: mouse lung cancer cells. It was found that PACE4 activity was not detected in normal cells but a great deal of PACE4 was produced in prostate cancer, colon cancer and lung cancer cells. It was also revealed that, in hepatic cancer cells, a certain aspartic enzyme distinct from PACE4 was present. In breast cancer cells, no PACE4 activity was detected.

The above finding strongly suggests that measurement of the activity of the present enzyme might be a key marker for recognizing the state of cancer. The present inventors measured PACE4 activity in plasma from patients suffering from cancer with the PACE4 screening system. As a result, the activity in plasma from patients suffering from cancer was measured to be 0.73 + 0.6 Unit (n=30) which was significantly higher than those from healthy individuals, 0.02 ± 0.1 Unit (n=6).

The enzyme prepared as described above or the protein fragments with anti-cancer activity that was a fragmented product with said enzyme, in order to maintain their activity at maximum, is preferably used freshly, or if stored, they are stored at 4°C and preferably used within 7 days after storage. Alternatively, they may be stored by lyophilization or in a liquid state together with an appropriate stabilizing agent such as human albumin, gelatin, salts, sugars or amino acids, or even it is possible to store them by freezing. For the purpose of inactivating contaminant infectious viruses, they may preferably be treated under suitable conditions, such as by heating at 65°C for 96 hours, in the lyophilized state from the viewpoint of safety. PACE4 as an active ingredient or the protein fragments with anti-cancer activity produced by said enzyme may be formulated into a medicament for inhibiting metastasis and growth of cancer in combination with the conventional appropriate excipients using the conventional procedures.

An effective dose of the medicament for inhibiting metastasis and growth of cancer comprising as an active ingredient PACE4 or the plasma protein fragments with anti-cancer activity produced by PACE4 may vary depending on, for instance, age, symptoms, or severity of patients and will be determined by physician's discretion. It is envisaged, however, that, for instance, around 30 to 150 mg per adult may be administered at once or divided in two portions. Most preferably, it is administered by bolus (single and large amount) or by intravenous drip. Optionally, it may be used in combination with other anti-cancer agents, which may be present, in a preferable embodiment, in a medicament for inhibiting metastasis and growth of cancer.

The plasminogen fragments derived from blood as used in Examples herein were confirmed for their safety by a toxicity test with a single intravenous administration in mice, a general pharmacological test in which effects on the respiratory and circulatory organs were examined in Beagle dog, and a virus inactivation test.

### Effects of the medicament

The medicament for inhibiting metastasis and growth of cancer comprising as an active ingredient the plasma protein-fragmenting enzyme or the plasma protein fragments produced by said enzyme of the present invention may suitably be used for clinically treating solid cancers, typically lung cancer and colon cancer.

As reported by O'Reilly et al., it has been revealed that human angiostatin prepared by treating human plasminogen with elastase potently inhibited growth of the vascular endothelial cells and growth of tumor, which is dependent on vascularization (O'Reilly et al., Nat. Med., vol. 2, p.689-692 (1996)). Cancer treatment with an inhibitor to vascularization draws much attention to researchers in that it induces less side effects and less drug resistance than the conventional chemotherapy. On the other hand, an inhibitor to vascularization must be kept administered as long as cancer exists and thus a problem is how to provide a great deal of the proteinaceous substance, angiostatin. For solving this problem, preparation of angiostatin using the genetic engineering technique or introduction of angiostatin gene into patients suffering from cancer are highlighted. Introduction of PACE4 into patients suffering from cancer may be an alternative means to solve this problem.

That is, 1. since starting material for preparing angiostatin, plasminogen, is present in a large amount within the living body, it is possible to efficiently produce angiostatin from plasminogen using a small amount of the enzyme of the present invention; 2. the enzyme of the present invention not only produces angiostatin but also inactivates plasmin, the activity of which promotes cancer development, to thereby enable more effective exertion of the angiostatin activity; 3. since the enzyme of the present invention does not function within the normal living body where a neutral pH range is maintained except for the external secretion system (stomach) but functions only at cancerous environment with a lower pH range, at which PACE4 exerts its activity, it is efficient and safe; 4. the enzyme of the present invention cleaves not only plasminogen but also other proteins such as fibronectin and tetranectin to produce fragments having an inhibitory activity to vascularization; and 5. therefore, via synergistic effects, the enzyme of the present invention can inhibit metastasis and growth of cancer, which is dependent on vascularization, more efficiently.

The present invention is explained in more detail by means of the following Examples so that it may be more fully understood, but it should not be construed to be limited thereto.

### Example 1 (Preparation of Enzyme Stock Solution)

Human prostate cancer cells (PC-3) were provided from Professor Nakanobu Kuwano at Kyushu University, Faculty of Medicine. PC-3 cells were maintained in RPMI-1640 medium supplemented with 10% fetal calf serum (FCS) (manufactured by Nissui Seiyaku K.K.). When the cells became confluent, the medium was replaced with RPMI-1640 free of FCS (hereinafter referred to as "serum free medium") and culture was continued for additional 1 to 2 days. A culture supernatant was recovered, centrifuged (3000 rpm × 20 minutes), and filtered (0.45 µm Milex HA: manufactured by Millipore) to give an enzyme stock solution.

### Example 2 (Confirmation of Enzymatic Activity)

The enzymatic activity of the stock solution was estimated, in accordance with Gately et al., by reacting the enzyme stock solution with plasminogen, separating the reaction solution by SDS-PAGE, and analyzing with immunoblot using anti-LBSI antibody to determine a degree to which plasminogen was degraded. Example 3 (Effects of pH on Fragmentation of Plasminogen by Culture Supernatant)

The culture supernatant prepared in Example 1 (100 µl), a solution of plasminogen (1 mg/ml, 100 µl) and buffer solutions at various pHs were mixed together at a ratio, 1:1:2. The mixture was incubated at 37°C and the enzymatic activity was determined as described in Example 2. The results are shown in Fig. 1 wherein distinct fragmentation patterns of plasminogen were apparent between pH ranges of more and less than 5.0. The arrow (→) indicates a bond corresponding to the plasminogen fragment from PACE reported by Gately et al.

### Example 4 (Identification of Plasminogen Fragments Produced by PACE4)

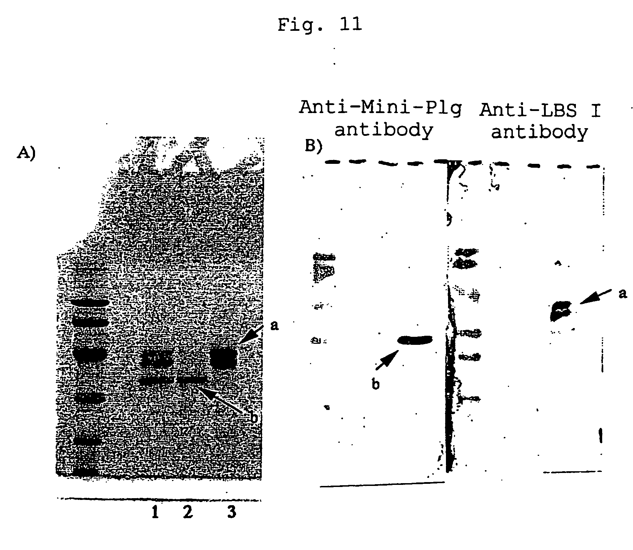

Among the reaction solutions in Example 3, the reaction solution at pH 4.0 was subjected to 12.5% SDS-PAGE and the proteins were then transferred to Immovilon membrane (manufactured by Millipore) in a conventional manner. To this was performed immunoblot analysis using rabbit anti-human plasminogen Lysine Binding Site I antibody and rabbit anti-human plasminogen Mini Plasminogen antibody. The results are shown in Fig. 11. As shown in Fig. 11, three bands were observed in electrophoresis under non-reducing condition. Among these, the band of 40, 43 kDa was reacted with rabbit anti-human plasminogen Lysine Binding Site I antibody whereas the band of 35 kDa to rabbit anti-human plasminogen Mini Plasminogen antibody. In Fig. 11, (a) lysine Sepharose bound fraction, and (b) lysine Sepharose unbound fraction.

### Example 5 (Cleavage Pattern at pH 4.0)

A reaction solution wherein plasminogen was fragmented under conditions described in Example 3 was subjected to 12.5% SDS-PAGE electrophoresis and the proteins were transferred to Immovilon membrane (manufactured by Millipore) by blotting. Then, dying with Amido Black and decoloration with purified water were performed and the band at around 40, 43 kDa and the band at around 35 kDa were excised. With an amino acid N-terminus analyzer (manufactured by Bio Applied), the N-terminal amino acid residue was determined for each band to identify 79Leu for the band of 40, 43 kDa and 480Pro for the band of 35 kDa. Based on their molecular weight, these 40 and 43 kDa fragments prepared by cleavage at pH 4.0 were estimated to comprise Kringles 1 to 4 and herein referred to as "PAN4" (PACE derived Angiostatin pH 4.0).

### Example 6 (Production of PACE4 in Various Cancer Cells)

Plasminogen was fragmented as described in Example 3 except that culture supernatants of human fibroblast cells, vascular endothelial cells from human umbilical vein, vascular endothelial cells from bovine aorta, human hepatic cancer cells (HepG2) and mouse lung cancer cells (LL/2) were used in place of PC-3 cells of Example 1. Analogous enzymes that fragmented plasminogen were found in supernatants from cancer cells other than PC-3.

### Example 7 (Screening System for PACE4)

Fig. 2 schematically illustrates procedures for screening PACE4 wherein anti-Mini Plasminogen antibody is used as an immobilized antibody whereas antibody directed to Kringles 1 to 3 of plasminogen is used as a labeled antibody. A predetermined amount of plasminogen and samples are mixed together and the mixture is added to this system. If plasminogen is cleaved at between Kringle 4 and Mini Plasminogen, a color development will become declined in proportion to a degree of the cleavage. An enzyme level is determined based on a rate of this decline in color development.

### (1) Preparation of samples

To 750 µl 0.1 M phosphate/citrate buffer (pH 3.0) were added 200 µl sample (culture supernatant or intermediate material obtained during purification) and then 5.0 µl plasminogen or Lys plasminogen at a final concentration 20 µg/ml and the mixture was reacted at 37°C for 1 hour. The reaction solution was diluted with a phosphate buffer containing 20 U/ml aprotinin and 1% BSA and served as samples for ELISA.

### (2) Procedures of ELISA

Anti-human LBSI antibody was dissolved in a dilution solution for antibody (phosphate buffer) at 20 µg/ml. Each 100 µl/well of the solution was added to a 96-well microtiter plate (IMMUNO MODULE MAXISORP F8, manufactured by Nunc) and the plate was incubated at 4°C overnight. The antibody solution was sucked from each well with a microtiter plate washer (manufactured by DIATECH, ULTRA WASH II). The plate was washed with PBS, added with 1% albumin 300 µl (Albumin Fraction V, Bovine, manufactured by Seikagaku Kogyo K.K.) and left to stand at 4°C overnight. After the albumin solution was sucked, the plate was washed with the above buffer (×3) and served as a well for determination. Separately, anti-mPlg antibody labeled with peroxidase was diluted in a phosphate buffer (pH 7.2) containing 0.05% Tween 20 at 20 ng HRP conjugate/ml.

To the well for determination was added the samples prepared as described above at 100 µl/well and the plate was left to stand at 37°C for 1 hour. The reaction solution was sucked from each well. The plate was washed with PBS (pH 7.2) containing 0.05% Tween 20 (x4), added with the solution (100 µl) of anti-mPlg antibody labeled with peroxidase and left to stand at 37°C for 30 minutes. The reaction solution was sucked from each well. The plate was washed with a phosphate buffer (pH 7.2) containing 0.05% Tween 20 (×4), added with a substrate solution 100 µl (OPD/H₂O₂) and left to stand at room temperature for 20 minutes with shading. To each well was added 2N sulfuric acid 100 µl to quench the reaction and absorbance at 490 nm was measured with a plate reader (WAKO Molecular Devices,

THRMO max microplate reader).

### (Results of ELISA)

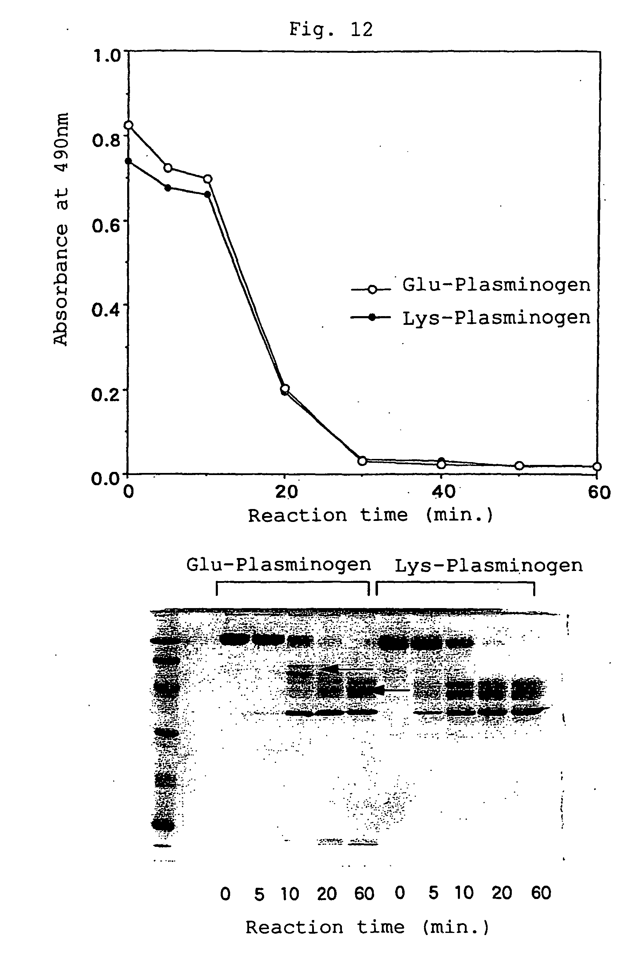

A serial dilution of a culture supernatant of PC-3 cells was measured by the system for enzyme determination according to the present invention. The results are shown in Fig. 12 wherein the open circle in the graph (upper) indicates those from plasminogen as a substrate whereas the closed circle from Lys plasminogen. The plasminogen-fragmenting enzyme in PC-3 culture supernatant cleaved both substrates with passage of time. As shown in Fig. 12, Lys plasminogen was cleaved more efficiently at earlier stage than Glu plasminogen but such a difference in a rate of cleavage between Lys and Glu plasminogens was diminished from 10 minutes onward. Fig. 12 also shows in lower part how plasminogen is cleaved with passage of time with electrophoretogram.

The above results show that PACE4 cleaves plasminogen so that Mini Plasminogen is firstly released and the Kringle domains follow. Also, the above results show that the same measurement was obtained from the substrates, both Glu and Lys plasminogens. As a matter of convenience, the present inventors restricted a substrate for use in the present determination system to Lys plasminogen and an enzymatic activity was defined such that one Unit was defined as an amount of enzyme that cleaves 10 µg Lys plasminogen in 1 minute.

### Example 8 (Purification of Plasminogen-degrading Enzyme)

The enzyme of the present invention (PACE4) was purified by a combination of various chromatographs including hydrophobic chromatography, anion exchange chromatography, gel filtration and hydroxyapatite.

The enzyme stock solution (5 L) prepared as described in Example 1 was diluted twice with 50 mM Tris buffer (pH 7.4). The solution was passed through CM-Sepharose 6B (Φ 5 × 150 mm, manufactured by Pharmacia) equilibrated with 50 mM Tris/50 mM NaCl buffer (pH 7.4) and then through heparin-Sepharose 4B (Φ 2.5 × 100 mm, manufactured by Pharmacia) equilibrated with the same buffer to prepare a crude solution of PACE4. To the crude solution was added 1 M ammonium sulfate and the mixture was left to stand overnight. The mixture was centrifuged at 6,000 rpm at 4°C and the supernatant was recovered.

The supernatant from centrifugation was filtered through filter paper with 25 µm of pore size (AP-25: manufactured by Amicon). The filtrate was treated with Phenyl-Sepharose Hiperformance (Φ 2.5 × 100 mm, manufactured by Pharmacia) equilibrated with 50 mM phosphate buffer (pH 7.2) containing 1 M ammonium sulfate. After washing with the same buffer, gradient elution was performed with 50 mM phosphate buffer (pH 7.2) followed by elution with 50 mM phosphate buffer (pH 7.2) containing 40% ethylene glycol.

Active fractions, which were eluted with 50 mM phosphate buffer (pH 7.2) and with the same buffer containing 40% ethylene glycol, were recovered and dialyzed against a large excess of 10 mM Tris buffer (pH 7.4). After dialysis, the solution was treated with Q-Sepharose Hiperformance (Φ 1.5 × 100 mm, manufactured by Pharmacia) equilibrated with 50 mM Tris buffer (pH 7.4) and gradient elution was performed with the same buffer containing 1 M NaCl. Active fractions were recovered, concentrated with ultrafiltration membrane (YM-10: manufactured by Amicon) and then gel filtration was performed by passing through Sephacryl S-200 (Φ 2.5 × 100 cm, manufactured by Pharmacia) equilibrated with 50 mM Tris/100 mM NaCl buffer (pH 7.4). Peaks eluted at a molecular weight of around 50,000 to 60,000 were collected and subjected to chromatography with Q-Sepharose Hiperformance (Φ 1.5 × 100 mm, manufactured by Pharmacia) under the above conditions to prepare active fractions. The obtained active fractions were finally dialyzed against 20 mM phosphate buffer (pH 7.4), passed through hydroxyapatite (Φ 2.5 × 100 mm, manufactured by BioRad) equilibrated with the same buffer and gradient elution was performed with 200 mM phosphate buffer (pH 7.4). The purified protein was detected as a band corresponding to a molecular weight of 42 to 45 kDa (under reduced condition). Fig. 4 shows SDS-PAGE after purification wherein PACE4 was identified as a protein with a molecular weight 42 to 45 kDa (under reduced condition in which sample was treated with mercaptoethanol).

### (Analysis and Identification of N-terminal Amino Acid Residue of PACE4)

The enzyme of the present invention prepared from PC-3 culture supernatant, after being transferred to GVDF membrane (Immovilon, manufactured by Millipore) by blotting, was sequenced with an amino acid sequencer (Applied Biosystem Model 477A protein sequencer). This revealed that the said enzyme had the N-terminal amino acid sequence LVRIPLHKFT, which was identical to that of Human Cathepsin D precursor as a result of homology search.

### Example 9 (Preparation of Plasminogen Fragments Using Enzyme of the Present Invention)

Plasminogen fragments produced by the enzyme of the present invention were prepared by incubating Lys plasminogen and the enzyme of the present invention and separating the reaction solution by lysine affinity chromatography. Lys plasminogen and the enzyme of the present invention were mixed together at a ratio 100:1 in a buffer of 50 mM Tris/0.15 M NaCl (pH 8.0) and reacted at 37°C for 3 hours. The reaction solution was passed through Lysine Sepharose 4B (Φ 10 × 15 mm) equilibrated with the same buffer and, after washing, elution was performed with the same buffer containing 0.1 M epsilon amino caproic acid. When plasminogen was used for preparing fragments, the reaction was at 37°C overnight followed by the same procedures. The obtained fraction was dialyzed against 0.1 M ammonium carbonate, lyophilized and stored at -80°C till use.

### Example 10 (Plasminogen Fragments Produced by the Enzyme of the Present Invention)

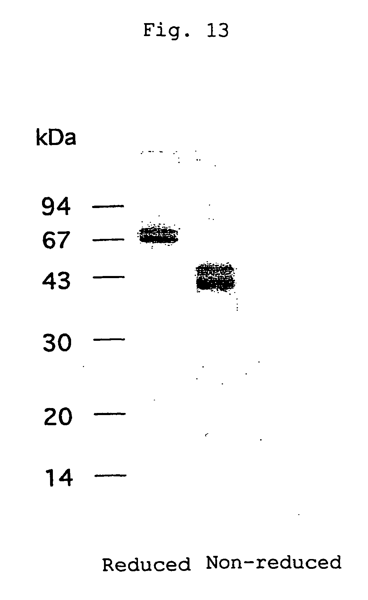

The plasminogen fragments comprising Kringles 1 to 4 prepared by the enzyme of the present invention were electrophoresed on 12.5% SDS polyacrylamide and dyed with Coomassie Blue. The results are shown in Fig. 13. Two bands corresponding to a molecular weight of 55 kDa and 63 kDa were observed with the plasminogen fragments treated with 2-mercaptoethanol (under reduced condition). Also, two bands corresponding to a molecular weight of 40 kDa and 43 kDa, molecular species after reduction, were observed with the plasminogen fragments not treated with 2-mercaptoethanol (under non-reduced condition).

The N-terminal amino acid residue was determined for the obtained plasminogen fragments. It was 77Lys in both cases where Lys plasminogen or plasminogen was used as a substrate.

### Example 11 (Inhibitory Activity of PACE4 to Growth and Metastasis of Cancer in Immunodeficient Animal)

### 1. Cancer cells

Lewis lung cancer cells, LL/2, were purchased from Dainippon Seiyaku K.K. whereas Lewis lung cancer cells, 3LL, were given from Cell Resource Center for Biomedical Research, Institute of Development, Aging and Cancer, Tohoku University. These cells were cultured for maintenance on RPMI-1640/High Glucose medium supplemented with 10% FBS (manufactured by Dainippon Seiyaku K.K.) at 37°C and 5% CO₂.

### 2. Mice

C57B1 mice of six weeks old were purchased from Kyushu Dobutsu K.K. whereas immunodeficient (Scid) mice were purchased from Charles River. These animals were adapted and bred in a sterile house for a week, with 4 to 5 animals being accommodated per cage, until experiment.

To immunodeficient mice of 6 weeks old were transplanted subcutaneously 10⁶ Lewis lung cancer cells (LL/2) at the back. The animals were bred for 14 to 17 days and cancer (primary cancerous focus) formed at the back was surgically removed. Based on size of the primary cancerous focus formed, the animals were divided into two groups: one with the primary cancerous focus of not more than 1200 mg (200 mg to 1200 mg) and the other of more than 1200 mg (1201 mg to 2500 mg). Each of these two groups was further divided into three groups: ones that received a high or low dose of the enzyme of the present invention (PACE4) and control. The animals were bred for 4 days after surgery and thereafter daily administered intraperitoneally with either 10 µg/animal or 2 µg/animal of PACE4 or 100 µl physiological saline for 10 days. Mice were dissected and the lung was weighed. The results are shown in Fig. 9 wherein (A) Group with the primary cancerous focus of not more than 1200 mg; (B) Group with the primary cancerous focus of more than 1200 mg; and the dot line indicates a weight of normal lung. As shown in Fig. 9, no significant difference was observed between PACE4 administration and control in case of the group with the primary cancerous focus of not more than 1200 mg. However, in the group with the primary cancerous focus of more than 1200 mg, an inhibitory activity to increase in a lung weight, i.e. inhibitory activity to growth of metastasized cancerous focus, was observed in a dose dependent manner as a result of PACE4 administration.

### Example 12 (Administration to Cancer-bearing Animal of Plasminogen Fragments Produced by Cleavage with PACE4)

To C57B1 mice of 6 weeks old were transplanted subcutaneously 10⁶ Lewis lung cancer cells (LL/2) at the back. The animals were bred for 14 to 17 days and cancer (primary cancerous focus) formed at the back was surgically removed when it weighed 300 to 1200 mg. The affected part was disinfected and sutured. Mice were bred for additional 14 days and thereafter intraperitoneally administered with 25 µg/animal of the plasminogen fragments produced by cleavage with PACE4 for 10 days. On Day 11, mice were dissected and the lung was removed and weighed. The lung from the control group weighed 0.44 ± 0.28 g (n=6) whereas the lung from the group administered with the plasminogen fragments weighed 0.23 ± 0.08 g (n=6), indicating that the administration of the plasminogen fragments significantly inhibited growth of LL/2 cells after metastasis to the lung. Example 13 (Test for Inhibitory Activity to Growth of Vascular Endothelial Cells)

A test for inhibitory activity to growth of vascular endothelial cells was performed as described by Gately et al. using Human Umbilical vein endothelial (HUV) cells (HUVEC; Sanko Jun-yaku K.K.). HUVEC cells were plated on a 24-well plate (Nunclone, manufactured by Nunc) at 12,500 cells/well and cultured with a culture medium attached to the kit overnight. The cells were added with 1, 10, 50, or 100 µg/ml of the plasminogen fragments or a physiological saline as a control and cultured at 5% CO₂, 37°C, for 72 hours. The cells were peeled off the well with trypsin/EDTA solution and counted with Coulter counter (manufactured by Coulter).

The activity of the plasminogen fragments was also estimated as described by O'Reilly et al. using bovine aorta endothelial (BAE) cells purchased from Sanko Jun-yaku K.K. BAE cells were cultured for maintenance on a 24-well plate (manufactured by Iwaki Glass K.K.) coated with gelatin in a DMEM basal medium containing 10% fetal calf serum (FCS), 3 ng/ml basic FGF (bFGF), 1% glutamine, 1% penicillin and 1% streptomycin at 5% CO₂. BAE cells were plated on a 24-well gelatin-coated plate at 12,500 cells/well and cultured on the basal medium deprived of bFGF for 24 hours. The culture medium was replaced with a culture medium in which the plasminogen fragments were diluted to 100 µg/ml in the basal medium that contained 5% FCS but was deprived of bFGF and the plasminogen fragments were reacted with the cells at 37°C for 20 minutes. A physiological saline was used as a negative control whereas angiostatin (Angiostatin, manufactured by Technoclone) was used as a positive control. After reaction, the basal medium containing 5% FCS and 2 ng/ml bFGF was added and the cells were cultured at 37°C for additional 72 hours. The cells were peeled off the well with trypsin/EDTA solution and counted with Coulter counter (manufactured by Coulter).

### (Inhibitory Activity to Growth of Vascular Endothelial Cells of Plasminogen Fragments Prepared by PACE4)

The plasminogen fragments (0 to 20 µg/ml) prepared by PACE4 were reacted with the vascular endothelial cells to prove that said fragments had no inhibitory activity to growth of the vascular endothelial cells. On the contrary, angiostatin purchased from Technoclone exhibited the inhibitory activity.

### Example 14 (Expression of PACE4 in E. coli (cDNA Preparation))

As described in Example 8, the N-terminal analysis of PACE4 revealed that the N-terminal amino acid sequence of PACE4 had a high homology with the amino acid sequence of cathepsin D, lysosomal proteinase. Cathepsin D per se had the similar activity to PACE4. Thus, it was estimated that cathepsin D was responsible for the activity of PACE4. Based on these facts, cDNA of PACE4 was isolated as described below.

### 1. Purification of mRNA from Human Prostate Cancer Cells PC-3

A whole RNAs were extracted from about 1.6 × 10⁷ cells of human prostate cancer cells (PC-3) in a conventional manner using ISOGEN solution (manufactured by Wako Jun-yaku K.K.). From the obtained whole RNAs, 27 µg mRNAs were purified with oligo(dT) column. The purified mRNAs together with a 10-fold amount of 3 M sodium acetate and a 2.5-fold amount of cold ethanol were stored at -80°C till use.

### 2. Synthesis and Amplification of cDNA

Using Super Script Preamplification System (GIBCO BRL), cDNA of PACE4 was synthesized from 1 µg of the purified mRNA using oligo(dT) as a primer in accordance with the manufacturer's instructions. Then, sense and antisense primers for amplifying a whole translation region of PACE4 gene were synthesized based on the nucleotide sequence of cDNA of cathepsin D precursor laid open public on data base and a desired gene was amplified with AmpliTaq (manufactured by PERKIN ELMER).

### 3. Determination of Nucleotide Sequence of PACE4 cDNA

Using TA cloning kit (Invitrogen), the amplified cDNA fragment was cloned into a plasmid vector in accordance with the manufacture's instructions. With the plasmid as a template, the cDNA was sequenced by the dideoxy terminating method using dideoxy nucleotides labeled with fluorescence. The determined nucleotide sequence and an amino acid sequence deduced therefrom were compared with the known sequences of human cathepsin D precursor to confirm an extremely high homology between them.

### Example 15 (PACE4 Activity in Plasma from Patients Suffering from Cancer)

### 1. Significance of Determining Activity of PACE4

As described in Example 6, PACE4 is scarcely released out of normal cells but a great deal is released out of certain cancer cells. This implies possibility that PACE4 might be a key marker for examining cancerous state. PACE4 has an extremely high homology with human cathepsin D precursor as described above and thus anti-PACE4 antibody also strongly responds to human cathepsin D precursor and its active form human cathepsin D. Therefore, results obtained by using anti-human cathepsin may also be applied to PACE4 and can be important information for investigating correlation between cancer and PACE4. This correlation has been reported primarily for breast cancer. It is generally recognized that a high antigen level of human cathepsin D is observed in breast cancer and elevation of human cathepsin D level in turn makes breast cancer be malignant. However, on the contrary, the results in Example 6 indicate that the PACE4 activity that fragments plasminogen is scarcely detected for breast cancer cells (MFC-7) while Lewis lung cancer and prostate cancer cells, which inhibit growth of distally metastasized cancerous focus, release a great deal of the PACE4 activity out of cells. Accordingly, in case of breast cancer, it is inferred that there is disagreement between the antigenic level and the PACE4 activity.

The PACE4 activity is determined by measuring with ELISA an amount of plasminogen fragments produced after fragmentation by PACE4. This determination is about 100- to 1000-fold more sensitive than the determination of cathepsin D wherein pigment level in hemoglobin fragments produced after cleavage is measured by absorbance or based on an amount of protein. Thus, a trace amount of cathepsin D present in blood cannot be determined by the conventional procedure. The present inventors applied the screening method for PACE4 as described in Example 7 to determination of the PACE4 activity in plasma from patients suffering from cancer and measured the PACE4 activity.

### 2. PACE4 Activity in Plasma from Patients Suffering from Cancer

PACE4 activity was measured as described in Example 7 for plasma from patients suffering from either breast cancer, hepatic cancer or lung cancer and for plasma from healthy individuals.

### (1) Measurement of PACE4 in Plasma from Patients Suffering from Cancer

Plasma 100 µl from patients suffering from cancer, a physiological saline 100 µl containing 1% EDTA and a pH-modifying solution 100 µl were added to a microcentrifuge tube and the mixture was incubated at 37°C overnight. The reaction solution was centrifuged at 15,000 rpm for 3 minutes. A supernatant was collected and used as a sample for determination. The sample was subjected to ELISA as described in Example 7 to measure the PACE4 activity. On the other hand, plasma 100 µl from healthy individual, PACE4/100 µl (0 to 50 Unit) and a pH-modifying solution 100 µl were added to a microcentrifuge tube and the mixture was incubated at 37°C overnight, followed by the procedures as described above. The obtained measurements were used to prepare a calibration curve. In this determination, endogenous plasminogen present in plasma was used as a substrate. Thus, endogenous plasminogen level was measured with ELISA for determination of plasminogen and made uniform.

### (2) Results

The enzyme activity in plasma from patients suffering from cancer was determined to be 0.73 ± 0.6 Unit (n=30) which was significantly higher than that from healthy individuals, 0.02 ± 0.1 Unit (n=6). Determination for respective cancer was as follows: breast cancer, 0.66 ± 0.03 Unit (n=6); hepatic cancer, 0.69 ± 0.03 Unit (n=8); lung cancer, 0.77 ± 0.04 Unit (n=10); other cancers, 0.70 ± 0.07 Unit (n=13). No significant difference in the enzyme activity was seen among different cancers.

### Example 16 (Production of Other Inhibitory Factor to Vascularization by PACE4)

Fibronectin was purified as described by Rouslahti et al. (Method Enzymol. vol. 82, p.803-831) using a resin with gelatin being a ligand. Fresh lyophilized plasma 500 ml was subjected to freezing-thawing. Resulting cryoprecipitate was dissolved in PBS 200 ml and the solution was left to stand at 4°C overnight. The solution was then centrifuged at 10,000 rpm to give transparent precipitate. The precipitate was dissolved in PBS at room temperature and the solution was passed through gelatin Sepharose 4B. After thorough washing, elution was performed with PBS containing 4M urea. The obtained fibronectin was subjected to gel filtration with Sephacryl HR500 (manufactured by Pharmacia), confirmed for its purity by SDS-PAGE and stored at -80°C till use.

Vitronectin and human hepatocyte growth factor (HGF) were purchased from Becton Dickinson and CalBiochem, respectively.

The above proteins were dissolved in 0.1M phosphate/citrate buffer and pH was adjusted to 4.0. To this solution was added the enzyme of the present invention at a ratio of PACE4 and the protein, 200:1 and the mixture was reacted at 37°C overnight. 0.5M phosphate buffer (pH 7.0) was added to the mixture to quench the reaction. The mixture was dialyzed against PBS and subjected to sterile filtration and the obtained filtrate was used as a sample.

### Example 17 (Fragmentation of Fibronectin and Inhibitory Activity to Vascularization)

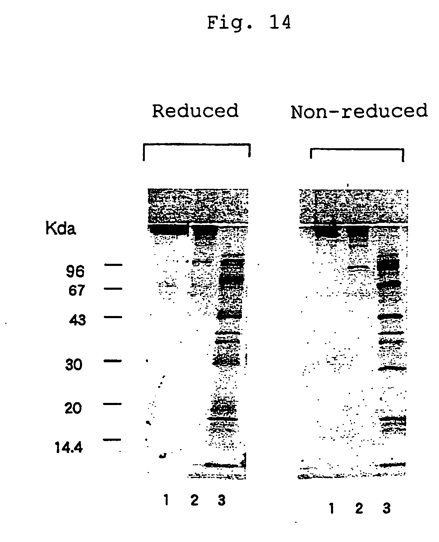

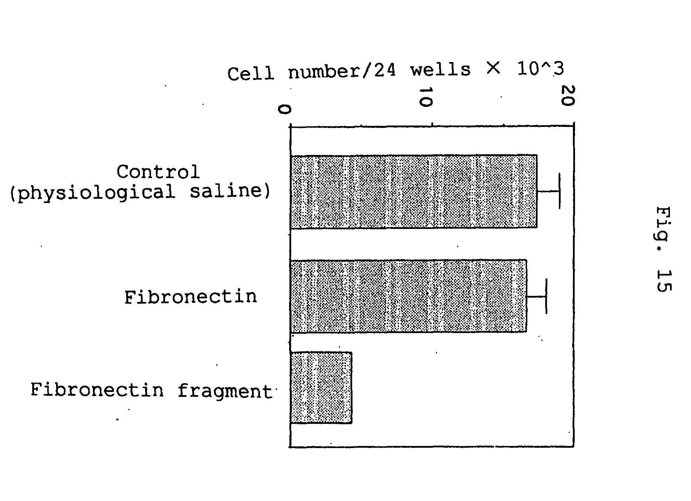

Effects on growth of the vascular endothelial cells were investigated for fibronectin and fibronectin fragments as described in Example 13. Fig. 14 shows electrophoretogram of the reaction mixture of PACE4 and fibronectin under indicated conditions wherein lane 1 indicates untreated fibronectin; lane 2, a reaction solution of fibronectin reacted at pH 4.0 for 12 hours; and lane 3, a reaction solution of fibronectin and PACE4 reacted at pH 4.0 for 12 hours. Fig. 15 shows results obtained by reacting the fragmented products of fibronectin by PACE4 with the vascular endothelial cells. As shown in Fig. 15, the fibronectin fragments produced by PACE4 fragmentation significantly inhibited growth of the vascular endothelial cells while fibronectin per se not fragmented by PACE4 exhibited no such inhibitory activity.

## Claims

1. In vitro Use of an aspartic enzyme having high homology to a cathepsin D precursor for producing plasma protein fragments having an inhibitory activity to metastasis and growth of cancer, wherein said enzyme is **characterized by** that:
(a) it has a molecular weight of about 45 kDa as measured by SDS electrophoresis under non-reduced condition;
(b) it has the N-terminal amino acid sequence LVRIPLHKFT; and
(c) it degrades plasma proteins at an acidic pH range of not more than pH 5.0 to produce plasma protein fragments having an inhibitory activity to metastasis and growth of cancer;
and wherein said plasma proteins to be fragmented are selected from the group consisting of plasminogen, vitronectin and human hepatocyte growth factor (HGF).

2. The use of claim 1 wherein said enzyme cleaves plasminogen at 73L-74F and/or 451 L-452P to produce fragments comprising Kringles 1 to 4 of plasminogen.

## Patentansprüche

1. Verwendung eines Asparaginenzyms in vitro, welches eine hohe Homologie zu einem Cathepsin D-Vorläufer hat, für die Produktion von Plasmaproteinfragmenten, die eine inhibitorische Aktivität auf Krebsmetastase und -wachstum haben, wobei das Enzym **dadurch gekennzeichnet ist, dass**:
(a) es ein Molekulargewicht von etwa 45 kDa hat, wie mit SDS-Elektrophorese unter nicht-reduzierender Bedingung gemessen;
(b) es die N-terminale Aminosäuresequenz LVRIPLHKFT hat; und
(c) es Plasmaproteine in einem sauren pH-Bereich von nicht mehr als pH 5.0 abbaut, um Plasmaproteinfragmente zu produzieren, die eine inhibitorische Aktivität auf Krebsmetastase und -wachstum haben;
und wobei die Plasmaproteine, die fragmentiert werden sollen, ausgewählt sind aus der Gruppe bestehend aus Plasminogen, Vitronectin und menschlichem Hepatocytenwachstumsfaktor (HGF).

2. Verwendung nach Anspruch 1, wobei das Enzym Plasminogen an 73L-74F und/oder 451L-452P spaltet, um Fragmente zu produzieren, die Kringles 1 bis 4 von Plasminogen umfassen.

## Revendications

1. Utilisation *in vitro* d'une enzyme aspartique présentant une homologie élevée avec un précurseur de la cathepsine D pour produire des fragments de protéines plasmatiques possédant une activité inhibitrice contre les métastases et la croissance d'un cancer, où ladite enzyme est **caractérisée en ce que** :
(a) elle a une masse moléculaire d'environ 45 kDa, mesurée par électrophorèse avec du SDS dans des conditions non réductrices ;
(b) elle a la séquence d'acides aminés N-terminale LVRIPLHKFT ; et
(c) elle dégrade les protéines plasmatiques au niveau d'une plage de pH acides de pas plus de pH 5,0 pour produire des fragments de protéines plasmatiques possédant une activité inhibitrice contre les métastases et la croissance d'un cancer ;
et où lesdites protéines plasmatiques à fragmenter sont choisies dans le groupe comprenant le plasminogène, la vitronectine et le facteur de croissance des hépatocytes humaines (HGF).

2. Utilisation selon la revendication 1, où ladite enzyme clive le plasminogène au niveau de 73L-74F et/ou de 451L-452P pour produire des fragments comprenant les kringles 1 à 4 du plasminogène.
